# EUROPÄISCHE PATENTANMELDUNG

(11) **EP 1 250 898 A1**
(43) Veröffentlichungstag der Anmeldung: **23.10.2002**
(21) Anmeldenummer: 01108607.1
(22) Anmeldetag: 05.04.2001
(51) Int. Cl.: A61F 2/44

(54) **Sytem von Bandscheibenprothesen**

(71) Anmelder: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(57) **Zusammenfassung**

System von Bandscheibenprothesen, das Standard- und Korrekturprothesen umfaßt. Die Korrekturprothesen dienen zum Ausgleich eines ventro-dorsalen Versatzes. Sie sind dadurch gekennzeichnet, daß an einer ihrer Deckplatten 15 diejenige Fläche (4'), über welche sie passend mit dem Prothesenkern (3') zusammenwirkt, im Vergleich mit den Standardprothesen gegenüber der Anschlußfläche (7) ventro-dorsal versetzt ist.

## Beschreibung

Es ist bekannt, beschädigte Bandscheiben durch Prothesen zu ersetzen, die aus zwei jeweils mit einem benachbarten Wirbelkörper zu verbindenden Deckplatten und einem Prothesenkern bestehen, wobei der Prothesenkern mit einer oder beiden Deckplatten über sphärische, komplementäre Gelenkflächen zusammenwirkt (EP-B 298 233). Die Deckplatten weisen Anschlußflächen auf, vermittelst deren sie mit den benachbarten Wirbelkörpern verbunden werden. Erwünscht ist, daß das Gelenkzentrum der Prothesen so angeordnet ist, daß die von der Prothese ermöglichten Bewegungsverhältnisse den natürlichen möglichst weitgehend gleichen und eine gleichmäßig verteilte Kraftübertragung zwischen den Wirbelkörpern und der Prothese stattfinden kann. Bei bekannten Prothesen sucht man diesem Ziel dadurch nahe zu kommen, daß das Gelenkzentrum in einer vorbestimmten räumlichen Beziehung zu den Anschlußflächen der Deckplatten angeordnet ist und die Deckplatten mit einem Rand versehen werden, der sich an die ventrale Randkante des zugehörigen Wirbelkörpers anlegt und dadurch die Relativlage der Deckplatte zum Wirbelkörper bestimmt (EP-B 560 140), oder daß ein Einsetzwerkzeug verwendet wird, das einen Anschlag am Wirbelkörper aufweist (EP-B 333 990). Dadurch wird eine stets gleiche Lage des Gelenkzentrums der Prothese im Verhältnis zu der ventralen Kante der Wirbelkörper erreicht. Bekannt ist es auch (EP-A 955 021), in einem System von Bandscheibenprothesen, das mehrere Klassen unterschiedlicher Größen umfaßt, Korrekturprothesen vorzusehen, die auf der einen Seite einer Größenklasse und auf der anderen Seite einer anderen Größenklasse angehören.

Durch die erwähnte räumlich vorbestimmte Beziehung des Gelenkzentrums zu den Anschlußflächen der Prothesen wird die gewünschte räumliche Beziehung zu den Wirbelkörpern nur dann erreicht, wenn die anatomischen Verhältnisse den bei der Konstruktion der Prothese vorausgesetzten Normverhältnissen entsprechen. Wenn aber beispielsweise aus anatomischen Gründen ein Gelenkzentrum ausnahmsweise eine andere Lage haben soll, muß die betreffende Prothese oder eine Deckplatte dieser Prothese abweichend von der normalen räumlichen Beziehung zum Wirbelkörper eingesetzt werden, was schwierig und riskant ist.

Ausgehend von dem oben an letzter Stelle genannten Stand der Technik liegt der Erfindung daher die Aufgabe zugrunde, für diese Fälle das Einsetzen der Prothese leichter und sicherer zu machen. Dies gelingt durch die Merkmale des Anspruchs 1 sowie vorzugsweise die Merkmale der Unteransprüche.

Vorausgesetzt wird ein System von Bandscheibenprothesen, das Standard- und Korrekturprothesen umfaßt. Die Standardprothesen sind in jeder Größenklasse einander gleich. Normalerweise sind mehrere Größenklassen vorhanden; jedoch ist dies nicht unbedingt erforderlich. Die Prothesen bestehen aus einem Prothesenkern und mindestens einer Deckplatte. Der Prothesenkern wirkt mit mindestens einer Deckplatte über Gelenkflächen zusammen. Vorzugsweise weist der Prothesenkern zwei Gelenkflächen auf gegenüberliegenden Seiten auf, über die er mit komplementären Gelenkflächen von zwei Deckplatten zusammenwirkt. Jedoch ist es auch möglich, daß eine der Deckplatten ungelenkig mit dem Prothesenkern über Halteflächen zusammenwirkt. Gelenkflächen und Halteflächen werden im folgenden und in den Ansprüchen zum Teil unter dem Begriff Kern-Passflächen zusammengefaßt. Zum Anschluß an einen Wirbelkörper besitzen die Deckflächen eine Anschlußfläche. Erfindungsgemäß ist in diesem Zusammenhang vorgesehen, daß die Korrekturprothesen mindestens eine Korrektur-Deckplatte aufweisen, deren Kern-Passfläche im Vergleich mit den Standardprothesen gegenüber der Anschlußfläche in ventro-dorsaler Richtung versetzt ist.

Auf diese Weise erreicht man, daß das Gelenkzentrum einer Prothese gegenüber demjenigen Wirbelkörper, auf dessen Seite die Korrektur-Deckplatte eingesetzt ist, gegenüber den Standardprothesen in ventro-dorsaler Richtung versetzt ist. Die Versetzung kann nach ventral oder nach dorsal geschehen, je nach Art und Einbaurichtung der Deckplatte. Wenn die Anschlußflächen der Korrektur-Deckplatten in bezug auf ihre medio-laterale Mittellinie symmetrisch ausgebildet sind, können sie wahlweise mit Zentrumsversatz nach ventral oder dorsal verwendet werden. Da überwiegend ein Versatz nach dorsal in Frage kommt, zeichnet sich eine wesentliche Ausführungsform der erfindungsgemäßen Korrekturplatte dadurch aus, daß das Gelenkzentrum nach dorsal versetzt ist, vorausgesetzt, daß man an der Deckplatte die dorsale Seite erkennen kann.

Es kommen Fälle vor, in denen die Bandscheibenprothese aufgrund starker Wirbelsäulenkrümmung oder aufgrund zwischen den betroffenen Wirbeln wirkender, hoher ventro-dorsaler Kräfte zu einem unerwünschten Versatz neigt. Dies gilt insbesondere für solche Prothesen, bei denen der Prothesenkern mit beiden Deckplatten über sphärische Gelenkflächen zusammenwirkt. Bei diesem Prothesentyp vermögen die Deckplatten unter seitlichen Relativkräften sich translatorisch ein wenig gegeneinander unter Schiefstellung des Prothesenkerns zu verschieben. Bei diesem Prothesentyp ist die Anwendung der Erfindung besonders vorteilhaft, weil die Korrekturplatten die Möglichkeit geben, das Gelenkzentrum der einen Deckplatte relativ verschoben zu dem Gelenkzentrum der anderen Deckplatte anzuordnen, wodurch der erwähnte Versatz kompensiert wird.

Den Durchmesser der Gelenkflächen wählt man innerhalb der Begrenzung der Deckplatte gerne so groß wie möglich, um die Flächenpressung gering zu halten. Bei bekannten Prothesen hat die an der Deckfläche ausgebildete Gelenkfläche eine nur wenig geringere ventro-dorsale Erstreckung als die Deckplatte selbst. In diesen Fällen kann der Versatz der Gelenkfläche gegenüber der Anschlußfläche nur unter der Voraussetzung stattfinden, daß für die Gelenkfläche ein kleinerer Durchmesser gewählt wird. Wenn man den Krümmungsradius der Gelenkfläche unverändert läßt, verringert sich bei der Durchmesserverringerung die Tiefe der in der Deckplatte vorgesehenen Gelenkfläche und damit ihre Fähigkeit, seitliche (parallel zur Ebene der Deckplatte) wirkende Kräfte zu übertragen. Dies mag in manchen Fällen akzeptabel sein, so daß diese Ausführung der Erfindung nicht ausgeschlossen wird. Bevorzugt wird jedoch eine Ausführung, bei welcher Durchmesser und Krümmungsradius der Gelenkfläche der Korrektur-Deckplatte übereinstimmend mit den Gelenkflächen gewählt werden, die einer anderen, kleineren Größenklasse angehören. Bei der kleineren Größenklasse wird nämlich nicht nur der Durchmesser der Gelenkflächen kleiner gewählt, sondern auch der Krümmungsradius. Die Tiefe dieser kleineren Gelenkflächen ist daher größer als die Tiefe einer Gelenkfläche der größeren Größenklasse, bei der lediglich der Durchmesser, nicht aber der Krümmungsradius verringert wurde.

Durch die Wahl einer Gelenkfläche (oder sonstigen Kern-Passfläche) schließt man zwar aus, daß die Korrektur-Deckplatte zusammen mit Prothesenteilen verwendet wird, die derselben Größenklasse angehören. Jedoch können statt dessen Prothesenteile verwendet werden, die der niedrigeren Größenklasse angehören, nach deren Vorbild die Gelenkfläche der Korrektur-Deckplatte gewählt wurde. Der Prothesenkern ist zweckmäßigerweise in allen Fällen ein Standard-Prothesenkern. Die zweite zu der Prothese gehörige Deckplatte ist ebenfalls zweckmäßigerweise eine Standard-Deckplatte. Jedoch kann, wenn ein besonders hoher Versatz der beiden Anschlußflächen der Prothesen zueinander gewünscht wird, auch die zweite Deckplatte eine Korrektur-Deckplatte sein, die allerdings gegenüber der ersten um 180° richtungsverdreht eingesetzt wird, so daß sich die Versatzbeträge beider Platten addieren. Nur wenn aus irgendwelchen Gründen das Gelenkzentrum der Prothese gegenüber beiden benachbarten Wirbeln nach ventral oder dorsal verschoben werden soll, verwendet man auf beiden Seiten Korrektur-Deckplatten in übereinstimmender Ausrichtung.

Bei den Korrektur-Deckplatten ergibt sich infolge der versetzten Anordnung der Kern-Passfläche gegenüber der Anschlußfläche auf einer Seite der Kern-Passfläche ein Abstand zwischen dieser und dem Plattenrand. Wenn dieser Teil der Fläche nicht benötigt wird, um eine möglichst große Anschlußfläche zur Verfügung zu stellen, kann die Platte auf dieser Seite gekürzt werden. Die Anschlußfläche ist dann in medio-lateraler Richtung ebenso breit wie die Standard-Deckplatten derselben Größenklasse, aber ihre Abmessung in ventro-dorsaler Richtung ist geringer und kann der kleineren Größenklasse entsprechen. Das kann Anpassungsvorteile ergeben in solchen Fällen, in denen die Wirbelkörper in medio-lateraler Richtung breiter und in ventro-dorsaler Richtung schmaler als normal sind.

Die Erfindung wird im folgenden näher unter Bezugnahme auf die Zeichnung erläutert, die vorteilhafte Ausführungsbeipiele veranschaulicht. Es zeigen:
- FIG. 1: einen Medianschnitt durch eine Standardprothese einer ersten Größenklasse,
- FIG. 2: eine Ansicht der Innenseite einer Deckplatte einer Standardprothese der ersten Größenklasse,
- FIG. 3: einen Medianschnitt durch eine Standardprothese einer zweiten Größenklasse,
- FIG. 4: eine Ansicht der Innenseite einer Deckplatte der Standardprothese gemäß FIG.3,
- FIG. 5: eine erste Ausführungsform einer Korrekturprothese im Medianschnitt,
- FIG. 6: eine Ansicht der Innenseite der zugehörigen Korrekturdeckplatte,
- FIG. 7: eine zweite Ausführungsform der Korrekturprothese im Medianschnitt,
- FIG. 8 und 9: Medianschnitte durch Standardprothesen unterschiedlicher Größenklassen einer abgewandelten Ausführungsform und
- FIG. 10 und 11: zwei Korrekturprothesen zu der abgewandelten Ausführungsform.

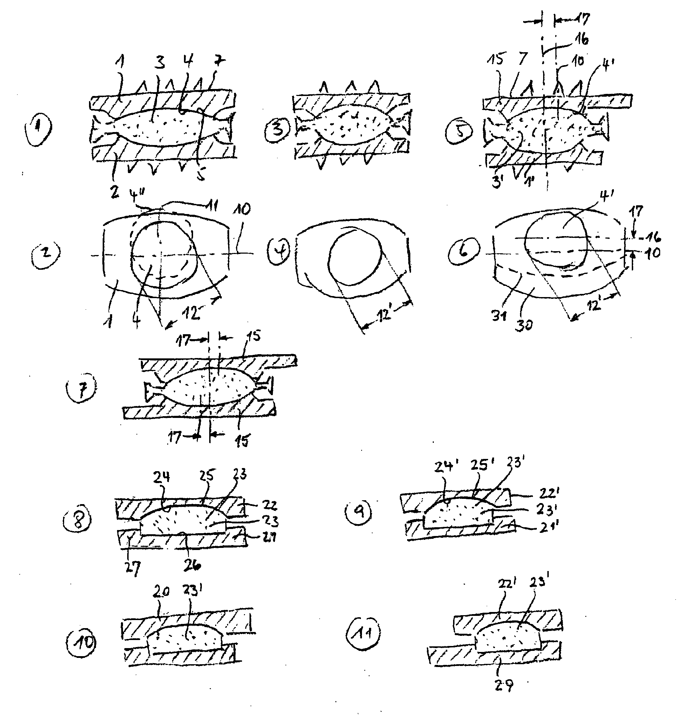

Die Standardprothesen gemäß FIG.1 bis 4 bestehen aus einer unteren Deckplatte 1, einer oberen Deckplatte 2 und einem Prothesenkern 3. Die Deckplatten bilden sphärische, konkave Gelenkflächen 4 und der Prothesenkern 3 zwei einander gegenüberliegende, identische, konvexe, sphärische Gelenkflächen 5, die zu denen der Deckplatten komplementär ausgebildet sind. Alle Maße der ersten Größenklasse (FIG.1 und 2) sind größer als die der zweiten Größenklasse (FIG.3 und 4). Außer den beiden dargestellten Größenklassen können weitere Größenklassen innerhalb des Systems vorhanden sein.

Die Komponenten der Prothese können aus Werkstoffen bestehen, die sich für Endoprothesen bewährt haben, beispielsweise Metall, Keramik, Polyethylen, wobei die Deckplatten 1, 2 vorzugsweise aus starrem Material (beispielsweise Metall) bestehen sollen und der Prothesenkern aus Polyethylen.

Die Deckplatten 1, 2 weisen eine Anschlußfläche 7 auf, die zur Verbindung mit der Stirnfläche eines Wirbelkörpers bestimmt ist. Sie kann mit nicht dargestellten Mitteln zur festen Verbindung mit dem Knochen ausgerüstet sein, beispielsweise Zähnen. Sie ist in ML-Richtung, die in FIG.2 durch die Linie 10 bezeichnet ist. ausgedehnter als in der VD-Richtung 11. Der Durchmesser 12 der Gelenkfläche 4 ist in den Standardprothesen so groß gewählt, wie es die Abmessung der Deckplatte in der Richtung 11 zuläßt. Diese Erläuterungen zu FIG.1 und 2 gelten ebenso für die zweite Größenklasse gemäß FIG.3 und 4.

Wie bekannt, sind derartige Prothesen dazu geeignet, die in Richtung der Wirbelsäule verlaufenden Kräfte und in gewissem Maße auch quer dazu verlaufende Kräfte von Wirbel zu Wirbel zu übertragen und gleichzeitig Schwenkbewegungen zu ermöglichen. Wenn sie zwischen Wirbeln eingesetzt werden, zwischen denen starke Querkräfte wirken oder sich eine starke Richtungsänderung vollzieht, wie dies beispielsweise zwischen dem letzten Lendenwirbel und dem Kreuzbein oft der Fall ist, kann sich eine Verschiebung der Deckplatten und der Wirbel ergeben. Diese kann durch Verwendung einer Korrekturprothese 14 ausgeglichen werden. Sie ist beispielsweise so aufgebaut, wie dies in FIG.5 oder in FIG.7 dargestellt ist.

Gemäß FIG.5 hat die Korrekturprothese eine obere Deckplatte 15, die als Korrektur-Deckplatte ausgebildet ist. Die Außenmaße ihrer Anschlußfläche 7 und damit die gesamten Flächenmaße gleichen denen der Standarddeckplatte 2 der ersten Größenklasse. Das Zentrum 16 der auf ihrer Innenseite vorgesehenen Gelenkfläche 4' ist gegenüber der Mittellinie 10 um einen Betrag 17 nach dorsal verschoben. Würde man dafür die Gelenkfläche 4 der Standarddeckplatte verwenden, so würde diese die in FIG.2 gestrichelt angegebene Lage 4" annehmen. Da diese teilweise über den Rand der Standardprothese hinaus führt, müßte diese an dieser Stelle mit einem Vorsprung oder einer Verbreiterung versehen werden oder müßte die Gelenkfläche entsprechend dem Standard-Randverlauf gekürzt werden. Beides ist zwar im Rahmen der Erfindung möglich, aber im allgemeinen nicht zweckmäßig. Bevorzugt wird daher die Ausführung gemäß FIG.6, bei welcher für die Gelenkfläche 4' diejenige der zweiten Größenklasse verwendet ist, deren Durchmesser 12' entsprechend geringer ist und daher innerhalb des Rands des Standardformats der Korrektur-Deckplatte 15 untergebracht werden kann. Passend zu dieser Gelenkfläche 4' ist die Korrektur-Deckplatte 15 mit einem Prothesenkern 3' und einer unteren Deckplatte 1' der zweiten Größenklasse kombiniert, wie dies in FIG.5 gezeigt ist. Vergleicht man in dieser Korrekturprothese die Lage der Gelenkmittellinie 16 mit der Lage der Anschlußfläche 7 der Korrektur-Deckplatte 15, so stellt man fest, daß diese nicht wie bei den Standardprothesen zusammenfallen, sondern um den Betrag 17 gegeneinander verschoben sind.

Statt mit einer Standarddeckplatte 1' der zweiten Größenklasse verbunden zu werden (FIG.5), kann in der Korrekturprothese als untere Deckplatte auch eine Korrektur-Deckplatte 15 um 180° verdreht eingesetzt werden (FIG.7). Der an der unteren Deckplatte erzielte Versatz 17 addiert sich zu dem an der oberen Deckplatte Stand findenden Versatz 17, so daß die Korrekturprothese gemäß FIG.7 im Vergleich mit derjenigen gemäß FIG.5 insgesamt den doppelten Versatz zur Verfügung stellt.

Wie gesagt, eignet sich die Erfindung besonders für den in den FIG.1 bis 7 dargestellten Prothesentyp, bei welchem der Prothesenkern 3 zwei einander gegenüberliegende Gelenkflächen 5 besitzt. Jedoch kann die Erfindung auch angewendet werden bei Prothesen des in FIG.8 bis 11 gezeigten Prothesentyps. FIG.8 und 9 zeigen Medianschnitte von Prothesen unterschiedlicher Größenklassen. Sie bestehen aus einer unteren Deckplatte 21, 21', einer oberen Deckplatte 22 bzw. 22' und einem Prothesenkern 23 bzw. 23'. Die obere Deckplatte 22, 22' und die Oberseite des Prothesenkerns 23, 23' gleichen denen des zuvor erläuterten Ausführungsbeispiels. Sie stellen eine gelenkige Bewegungsmöglichkeit längs des Gelenkflächenpaars 24, 25 zur Verfügung. Die Unterseite des Prothesenkerns 23 ist flach ausgeführt. Die untere Deckplatte 21, 21' bzw. deren Kern-Passfläche 26 mit Rand 27 ist lediglich zum Halten des Prothesenkerns 23, 23' ausgebildet.

Gemäß FIG.10 ist die obere Deckplatte 20 als Korrektur-Deckplatte gemäß den Erläuterungen zu FIG.5 und 6 ausgebildet. Sie gehört der ersten, größeren Größenklasse an. Sie ist zusammengesetzt mit einem Prothesenkern 23' und einer unteren Deckplatte 21' der zweiten, kleineren Größenklasse.

Statt dessen ist es auch gemäß FIG.11 möglich, die untere Deckplatte 29 als Korrekturplatte zu verwenden. Sie gehört der ersten Größenklasse an und ist zusammengesetzt mit einem Prothesenkern 23' und einer oberen Deckplatte 22' der zweiten Größenklasse. Wie in den oben erläuterten Beispielen wird dadurch ein Versatz 17 des Gelenkzentrums gegenüber der Anschlußfläche der Korrektur-Deckplatte erzielt.

In den Erläuterungen wurde einfachheitshalber der Versatz auf den Mittelpunkt der Anschlußfläche bezogen, der bei den Standardprothesen mit dem Gelenkzentrum der Prothese zusammenfällt. Es versteht sich aber, daß man sich bei der Feststellung des Versatzes auf jeden beliebigen Punkt der Anschlußfläche beziehen kann, wobei dessen Relativlage zum Gelenkzentrum einerseits bei einer Standarddeckplatte und andererseits bei Verwendung einer Korrektur-Deckplatte zu vergleichen ist.

Wie man in FIG.6 sieht, ist der Randstreifen 30 der Korrektur-Deckplatte 15 zur Unterbringung der Gelenkfläche (4') nicht erforderlich. Wenn er auch nicht im Hinblick auf eine erwünschte Größe der Anschlußfläche 7 benötigt wird, kann er wegfallen. Die Deckplatte 15 und deren Anschlußfläche 7 ist dann beispielsweise gemäß der strichpunktierten Linie 31 begrenzt.

## Patentansprüche

1. System von Bandscheibenprothesen, das Standard- und Korrekturprothesen umfaßt, wobei die Standardprothesen in mindestens einer Größenklasse jeweils untereinander gleich sind und aus einem Prothesenkern (3, 3', 23, 23') und mindestens einer Deckplatte (1, 1', 2, 2', 21, 21', 22, 22') bestehen, die eine mit dem Prothesenkern (3, 3', 23, 23') zusammenwirkende Kern-Passfläche (4, 4', 24, 26) und eine zum Anschluß an einen Wirbelkörper (9) bestimmte Anschlußfläche (7) aufweist, **dadurch gekennzeichnet, daß** die Korrekturprothesen (14) eine Korrektur-Deckplatte (15, 20, 29) aufweisen, deren Kern-Passfläche (4', 28, 26) im Vergleich mit den Standardprothesen gegenüber der Anschlußfläche (7) in ventro-dorsaler Richtung versetzt ist.

2. System nach Anspruch 1, **dadurch gekennzeichnet, daß** an die Korrektur-Deckplatten (15, 20, 29) eine dorsale Seite erkennbar ist und die Kern-Passfläche (4', 26, 28) gegenüber der Anschlußfläche (7) nach dorsal versetzt ist.

3. System nach Anspruch 1 oder 2, **dadurch gekennzeichnet, daß** der Durchmesser (12') der Kern-Passfläche (4', 26, 28) der Korrektur-Deckplatten (15, 20, 29) geringer ist als der der Standarddeckplatten.

4. System nach Anspruch 3, **dadurch gekennzeichnet, daß** es eine erste Größenklasse mit Standarddeckplatten (1, 2, 21, 22), deren Kern-Passflächen (4, 24) einen ersten Durchmesser (12) aufweisen, und eine zweite Größenklasse mit Standarddeckplatten (1', 2', 21', 22') umfaßt, deren Kern-Passflächen (4', 24', 26) einen zweiten Durchmesser (12') aufweisen, der kleiner als der erste Durchmesser (12) ist, und daß die erste Größenklasse Korrektur-Deckplatten (15, 20, 29) umfaßt, deren Kern-Passflächendurchmesser (12') dem Kern-Passflächendurchmesser (12') der Standarddeckplatten (1', 2', 21', 22') der zweiten Größenklasse gleichen.

5. System nach einem der Ansprüche 1 bis 4, **dadurch gekennzeichnet, daß** es Korrekturprothesen umfaßt, die aus zwei gegensinnig zueinander angeordneten Korrektur-Deckplatten (15) und einem Prothesenkern (3') bestehen.

6. System nach einem der Ansprüche 1 bis 5, **dadurch gekennzeichnet, daß** die Kern-Passflächen (4, 24, 4', 24') sphärische Gelenkflächen sind.

7. System nach einem der Ansprüche 4 bis 6, **dadurch gekennzeichnet, daß** es Korrektur-Deckplatten (15) umfaßt, deren Breite in ML-Richtung (10) der Breite der Standarddeckkplatten (1, 2, 21, 22) in derselben Größenklasse gleicht und deren Abmessung in der VD-Richtung (11) geringer ist als diejenige der Standarddeckplatten (1, 2, 21, 22) in derselben Größenklasse.
